# EUROPEAN PATENT APPLICATION

(11) **EP 3 329 922 A1**
(43) Date of publication of application: **06.06.2018**
(21) Application number: 16830603.3
(22) Date of filing: 29.07.2016
(51) Int. Cl.: A61K 31/7004, A61K 31/715, A61P 1/18, A61P 3/08, A61P 3/10, A61P 5/50, A61P 9/00, A61P 9/12, A61P 29/00, A61P 37/06, A61P 43/00

(54) **GLP-1 SECRETAGOGUE**

(30) Priority: 29.07.2015 JP 2015149634
(71) Applicant: Educational Foundation Jichi Medical University, Tokyo 102-0093 (JP); National University Corporation Hokkaido University, Sapporo-shi, Hokkaido 060-0808 (JP); Matsutani Chemical Industry Co., Ltd., Itami-shi, Hyogo 664-8508 (JP)
(72) Inventor: YADA, Toshihiko, Shimotsuke-shi Tochigi 329-0498 (JP); IWASAKI, Yusaku, Shimotsuke-shi Tochigi 329-0498 (JP); HARA, Hiroshi, Sapporo-shi Hokkaido 060-0808 (JP); HIRA, Tohru, Sapporo-shi Hokkaido 060-0808 (JP); KISHIMOTO, Yuka, Itami-shi Hyogo 664-8508 (JP); MINAMI, Machiko, Itami-shi Hyogo 664-8508 (JP)
(74) Representative: Schiener, Jens
(86) International application number: PCT/JP2016/072269
(87) International publication number: WO 2017/018500

(57) **Abstract**

An object of the present invention is to provide a GLP-1 secretagogue which is an incretin hormone-related drug relatively inexpensive, excellent in safety, and capable of promoting GLP-1 secretion without containing sucrose as an essential constituent. The object is achieved by a GLP-1 secretagogue characterized by containing D-psicose as an active ingredient.

## Description

### Technical Field

The present invention relates to an incretin-hormone GLP-1 secretagogue (GLP-1 secretion promoting agent or substance) useful for treating impaired glucose tolerance, preventing diabetes, or the like.

### Background Art

Glucagon-like peptide-1 (GLP-1) is one of incretin hormones, secreted from the digestive tract upon food ingestion, and has an action of promoting insulin secretion from the pancreas. In response to the influx of a nutrient into a lumen of the digestive tract, GLP-1 is secreted from L-cells, which are one type of endocrine cells of the digestive tract. GLP-1 then binds to a GLP-1 receptor on the β cell surface of the pancreas, promoting the insulin secretion from the inside of the β cell. It has been confirmed in animals that GLP-1 has actions such as: suppressing the secretion of a hormone glucagon, which increases the blood sugar level; protecting the pancreatic β cells; and promoting the β cell growth. Other actions of GLP-1 include such actions as cardioprotection, increasing cardiac output, alleviating hypertension, weakening inflammatory immune response, and delaying the discharge of ingested food from the stomach. A substance having an action of promoting GLP-1 secretion is quite useful.

On the other hand, according to "DIABETES ATLAS Sixth edition, 2014 UPDATE" summarizing the worldwide diabetes-related surveys, the worldwide diabetes population explosively continues increasing, and the number of patients with diabetes as of 2014 rises to 386.70 million (prevalent rate: 8.3%). The International Diabetes Federation (IDF) has predicted that if no effective countermeasure is taken, the number will increase to 591.90 million in 2035. In other words, it is apparent that diabetes is a worldwide serious disease.

In "Guideline for the Diagnosis of Diabetes Mellitus" of "Evidence-based Practice Guideline for the Treatment for Diabetes in Japan 2013" published by the Japan Diabetes Society, diabetes should be diagnosed based on the presence or absence of chronic hyperglycemia in addition to the symptoms and so forth. The presence or absence of hyperglycemia is determined based on a combination of the fasting blood sugar level and the 75 g oral glucose tolerance test (OGTT) 2-hour value, and also determined based on the HbAcl (NGSP) value ≥ 6.5%. Moreover, the fasting blood sugar level of 126 mg/dl or more is classified into the diabetic range, and that of 110 to 126 mg/dl is classified into the borderline range. Meanwhile, the OGTT 2-hour value of 200 mg/dl or more is classified into the diabetic range, and that of 140 to 200 mg/dl is classified into the borderline range. While the American Diabetes Association and the WHO distinguish between IFG (impaired fasting glucose) defined by the fasting blood sugar level and IGT (impaired glucose tolerance) defined by the OGTT 2-hour value, the Japan Diabetes Society calls the two "borderline type" collectively. Since this "borderline type" progresses to the "diabetic type" if no countermeasure such as treatment is taken at all, an appropriate treatment or countermeasure is required.

Recently, in the treatments against these "borderline type" and "diabetic type" diabetes-related diseases, attention has been focused on drugs related to incretin hormones: glucose-dependent insulinotropic polypeptide (GIP) and glucagon-like peptide-1 (GLP-1). Particularly, Japanese are said to be low in insulin secretion ability, especially insulin secretion ability after meal. So far, a therapeutic method for surely lowering the blood sugar level by injecting insulin has been commonly adopted. However, since insulin has a risk of excessively lowering the blood sugar level, attention has been focused on the aforementioned incretin hormone-related drugs by which the blood sugar level is not excessively lowered.

GIP is secreted by a stimulus to K cells mainly present in the upper intestinal tract. GLP-1 is, as described above, an incretin hormone secreted by a stimulus to L-cells mainly present in the lower intestinal tract. Incretin hormones act on the pancreas to promote insulin secretion in a hyperglycemia state, but do not promote insulin secretion in a non-hyperglycemia state. Hence, the risk of causing hypoglycemia is small. On the other hand, incretin hormones however have such a disadvantage that the hormones are rapidly degraded by an incretin-degrading enzyme (DPP-4). For this reason, a "DPP-4 inhibitor" capable of suppressing the incretin degradation and an "incretin hormone analogue" less susceptible to the DPP-4 action have been developed, and these are greatly expected to have less side effects. Nevertheless, both the incretin hormone-related drugs are not present in nature; hence, the cost for the productions is not inexpensive.

As other incretin hormone-related drugs than the aforementioned DPP-4inhibitor and incretin hormone analogue drug, disaccharide-degrading enzyme inhibitory drugs (a-GI agents) such as acarbose, voglibose, and miglitol have also been known. However, these are synthesized and not inexpensive. Moreover, although the drugs can be orally ingested together with daily foods, the prescriptions by doctors are required.

Further, since these conventional α-GI agents are antagonistic disaccharide-degrading enzyme inhibitors, the inhibitory actions vary, so that constant effects are not always obtained. To solve this problem, a drug has been proposed which is characterized in that the drug contains uncompetitive sucrose-degrading enzyme inhibitors L-arabinose, D-xylose, and/or D-tagatose instead of the antagonistic inhibitor, and also contains sucrose as an active ingredient or is ingested together with a food containing sucrose (Patent Literature 1). However, this drug is characterized by containing sucrose as an essential active ingredient, so that sucrose is intrinsically essential therein even though the intake of sucrose should be restricted for diabetes patients. Accordingly, extreme cares have to be taken for the usage, causing inconvenience.

### Citation List

### Patent Literature

Patent Literature 1: Japanese Patent Application Publication No. 2013-63947

### Non Patent Literature

Non Patent Literature 1: Tech. Bull. Fac. Agr. Kagawa Univ., Vol. 58, 27-32, 2006

### Summary of Invention

### Technical Problem

In view of the above-described conventional techniques, an object of the present invention is to provide a GLP-1 secretion promoter which is an incretin hormone-related drug relatively inexpensive, excellent in safety, and capable of promoting GLP-1 secretion without containing sucrose as an essential active ingredient or without administering sucrose separately.

### Solution to Problem

The present inventors have conducted various studies to achieve the object. As a result, the inventors unexpectedly found that D-psicose promotes GLP-1 secretion without requiring sucrose.

D-psicose has been known as an α-GI agent (Non Patent Literature 1). Hence, initially, the inventors have presumed that, like L-arabinose, D-xylose, or D-tagatose which are known as sucrose-degrading enzyme inhibitors, D-psicose would not promote GLP-1 secretion, either, if sucrose is not ingested at the same time. In addition, although the details will be described later, in the GLP-1 secretion experiment with murine large intestine-derived GLP-1 producing cells, D-psicose did not secrete GLP-1 unlike an indigestible dextrin (positive control) known to secrete GLP-1. From this experimental result, it cannot be predicted at all that D-psicose promotes GLP-1 secretion without containing sucrose as an essential active ingredient or without ingesting sucrose or a food containing sucrose at the same time. Thus, the fact that orally ingesting only D-psicose by a mammal can promote GLP-1 secretion is a surprising finding. Further, GIP mentioned above is known as an incretin hormone capable of promoting insulin secretion like GLP-1, but GIP is known to activate the lipid synthesis system and induce lipid accumulation unlike GLP-1. Meanwhile, the GLP-1 secretagogue of the present invention has been found not to promote GIP secretion.

Accordingly, the present invention has been completed based on the above-described findings, and includes the following [1] to [6].
[1] A GLP-1 secretagogue comprising D-psicose as an active ingredient.
[2] The GLP-1 secretagogue according to [1], wherein the GLP-1 secretagogue does not comprise sucrose.
[3] The GLP-1 secretagogue according to [1] or [2], wherein the GLP-1 secretagogue is administered during fasting.
[4] The GLP-1 secretagogue according to any one of [1] to [3], wherein the active ingredient D-psicose is administered at a single dose of at least 5 g.
[5] The GLP-1 secretagogue according to any one of [1] to [4], wherein the GLP-1 secretagogue is administered without ingesting sucrose or a food containing sucrose.
[6] A GLP-1 secretagogue composition comprising as active ingredients:
   the GLP-1 secretagogue according to any one of [1] to [5]; and
   a water-soluble dietary fiber.

### Advantageous Effects of Invention

The GLP-1 secretagogue of the present invention contains D-psicose as an active ingredient. The eating experience and safety of D-psicose have been acknowledged, and a side effect as observed for drugs is not exhibited. Sucrose is not contained as an essential ingredient. Hence, the GLP-1 secretagogue of the present invention is very easy to use and can promote GLP-1 secretion in mammals conveniently. Moreover, although D-psicose has 0 kilocalories, the sweetness is approximately 70% of that of sugar. The GLP-1 secretagogue of the present invention has an excellent GLP-1 secretion ability without requiring sucrose as an essential ingredient. Thus, the GLP-1 secretagogue of the present invention is useful in that the intake calorie can be greatly reduced in comparison with D-tagatose having a calorific value of 2 kilocalories/g and requiring sucrose for GLP-1 secretion.

### Brief Description of Drawings

Fig. 1 is a graph showing the result of a GLP-1 secretion experiment with 10 mM, 20 mM, and 40 mM solutions of D-psicose or a 10 mM solution of an indigestible dextrin (Fibersol-2) in murine large intestine-derived GLP-1 producing cells.
Fig. 2 shows graphs for illustrating the active GLP-1 concentration (a), the total GLP-1 concentration (b), and the total GIP concentration (c) in the portal veins over time after D-psicose was orally administered at a dose of 1 g per kg of fasting mice.
Fig. 3 shows graphs for illustrating the active GLP-1 concentration (a) and the total GIP concentration (b) in the portal blood 30 minutes after saline, D-psicose at 0.3 g/kg or 1.0 g/kg, D-tagatose at 1.0 g/kg, or D-glucose at 1.0 g/kg was orally administered into mice.
Fig. 4 is a graph for illustrating the effect of a GLP-1 receptor inhibitor (Exendin-9, Ex-9) on the ingestion suppressive effect by orally administering D-psicose at 1 g/kg using fasting mice. Specifically, Ex-9 (200 mmol/kg) or saline was intraperitoneally administered to the fasting mice, and immediately thereafter saline or D-psicose at 1 g/kg was orally administered. The graph shows the amount of diet ingested in terms of energy (kcal) over time from 30 minutes to 6 hours after the administration.
Fig. 5 is a graph for illustrating the amount of diet ingested over time from 1 hour to 6 hours after D-psicose or D-tagatose each at 1 g/kg was orally administered to mice, the amount being shown as a relative value to the amount of diet ingested when saline was orally administered.
Fig. 6 shows graphs for illustrating the blood glucose concentration (a) and the total GLP-1 concentration (b) over time from 0 minutes to 240 minutes after D-psicose, an indigestible dextrin (Fibersol-2), and a dextrin (the product manufactured by Matsutani Chemical Industry Co., Ltd.: "Pinedex #2") were administered each at 0.2 g/kg to SD rats.
Fig. 7 is a graph showing the result of calculating an area under blood concentration - time curve (AUC) by quantifying each total GLP-1 in blood after 200 ml of water or D-psicose at 5 g/200 ml, 10 g/200 ml, or 15 g/200 ml was ingested by humans, and 15 minutes, 30 minutes, 60 minutes, 120 minutes, and 180 minutes thereafter.

### Description of Embodiments

A drug of the present invention promotes GLP-1 secretion and is quite useful for alleviating various physiological functions, diseases, and symptoms through the GLP-1 function: treating impaired glucose tolerance, preventing and treating diabetes, and the like.

As an active ingredient D-psicose of the GLP-1 secretagogue of the present invention, any conventionally known D-psicose can be used regardless of the degree of purification. Such D-psicose includes: extracts from plants such as *Itea*; isomerization products from D-glucose and D-fructose as raw materials by alkali isomerization methods (for example, "Rare Sugar Sweet" manufactured by Matsutani Chemical Industry Co., Ltd.); isomerization products from D-glucose and D-fructose as raw materials by enzymatic methods utilizing enzymes (such as isomerases and epimerase) obtained from microorganisms or recombinants thereof (for example, "Astraea Allulose" manufactured by Matsutani Chemical Industry Co., Ltd.); and the like. These can be obtained relatively easily.

In order for the GLP-1 secretagogue of the present invention to exhibit the effects, it is important that D-psicose should be orally ingested. In this event, the state where sucrose is co-present in the intestines is not essential. In other words, it is not necessary that the GLP-1 secretagogue of the present invention be incorporated into a food containing sucrose, and it is not necessary that the GLP-1 secretagogue of the present invention be ingested together with sucrose or a food containing sucrose at the same time or administered after these ingestions, either.

The form of the GLP-1 secretagogue of the present invention is not particularly limited, and any form can be adopted, for example, tablet, granule, powder, capsule, gel, or sol. Moreover, the GLP-1 secretagogue of the present invention can be formulated according to known methods. The active ingredient D-psicose can be mixed with a pharmaceutically acceptable carrier such as starch or carboxymethyl cellulose, and further a stabilizer, an excipient, a binder, a disintegrant, or the like may be added thereto as necessary.

The GLP-1 secretagogue of the present invention is administered such that the amount of the active ingredient D-psicose is preferably at least 0.07/kg body weight per administration, more preferably 0.07 to 2.0 g/kg body weight per administration, and furthermore preferably 0.1 to 0.6 g/kg body weight per administration.

The GLP-1 secretagogue of the present invention is preferably ingested during fasting. Specifically, the GLP-1 secretagogue of the present invention is ingested preferably at least five minutes before meal. Further specifically, the GLP-1 secretagogue of the present invention is ingested more preferably three hours after the previous meal and at least ten minutes before meal.

The present invention provides a GLP-1 secretagogue composition characterized by further containing a water-soluble dietary fiber as an active ingredient in addition to the above-described GLP-1 secretagogue.

The water-soluble dietary fiber includes high-viscosity dietary fibers such as pectin, konjak mannan, alginic acid, guar gum, and agar; or low-viscosity dietary fibers such as indigestible dextrins, polydextrose, and guar gum degradation products.

Among these, indigestible dextrins, guar gum hydrolysates, and polydextrose are particularly preferable from the viewpoint of the effects.

Above all, low-viscosity dietary fibers are preferable from the viewpoints of handling and the short transit time to the large intestine. The low-viscosity water-soluble dietary fibers mean dietary fiber materials containing 50% by mass or more of dietary fibers and soluble in water at normal temperature to form low-viscosity solutions, which exhibit a viscosity of 20 mPas or less in the form of roughly 5% by mass of an aqueous solution. The low-viscosity dietary fibers more specifically include indigestible dextrins, guar gum hydrolysates, polydextrose (for example, Litesse and the like), hemicelluloses-derived products, and the like.

The indigestible dextrins are produced by: degrading various starches, for example, potato starch, tapioca starch, corn starch, wheat flour starch, or the like, by heating at 130°C or more; further hydrolyzing the resultant with an amylase; and as necessary decolorizing and desalting the hydrolysate according to conventional methods. The dietary fibers have an average molecular weight of approximately 500 to 3000, preferably 1400 to 2500, and further preferably around 2000. The glucose residues of the dextrins are linked by α-1,4, α-1,6, β-1,2, β-1,3, and β-1,6-glycosidic bonds, a portion of the reducing end is levoglucosan (1,6-anhydro-glucose), and the branch structure is well developed. The indigestible dextrins are commercially available under product names of "Nutriose" (manufactured by Roquette Group), "Pine Fibre," and "Fibersol-2" (manufactured by Matsutani Chemical Industry Co., Ltd.) ("*Shokuhin Shinsozai Forum* (New Food Ingredient Forum" NO.3 (1995, edited by Japanese Council for Advanced Food Ingredients)).

The guar gum hydrolysates are obtained by hydrolyzing guar gum with enzymes. The properties are normally low in viscosity, and soluble in cold water, and aqueous solutions thereof are neutral, colorless, and transparent. The guar gum hydrolysates are commercially available under product names of "Sunfiber" (Taiyo Kagaku Co., Ltd.) and "Fibaron" (Dainippon Pharmaceutical Co., Ltd.).

The hemicellulose-derived products are produced normally by purifying alkali-extracted products of the corn outer skins. Although the average molecular weight is as high as approximately 200,000, the viscosity of the 5% aqueous solution is as low as approximately 10 cps. The hemicellulose-derived products dissolve in water to form transparent liquids. The hemicellulose-derived products are commercially available under a product name of "Cellace" (Nihon Shokuhin Kako Co., Ltd.).

The polydextrose (Litesse) is obtained by polymerizing glucose and sorbitol in the presence of citric acid by heating at the hydraulic pressure, and purifying the polymer. The polydextrose is soluble in water and low in viscosity. The polydextrose is commercially available "Litesse" (Pfizer Inc.).

Among these low-viscosity water-soluble dietary fibers, the indigestible dextrins are the most effective and preferable.

As the active ingredient of the GLP-1 secretagogue of the present invention, D-psicose can be used alone. In a case where the GLP-1 secretion is desired to last for a long period, an indigestible dextrin is preferably used in combination. This is because the indigestible dextrin exhibits the effect of promoting GLP-1 secretion at a timing later than D-psicose, so that the combination makes it possible to keep the GLP-1 secretion for a long period.

The GLP-1 secretagogue of the present invention and the indigestible dextrin are incorporated at a mass ratio of preferably 1:0.1 to 1:100, more preferably 1:0.5 to 1:50.

### Examples

Hereinafter, excellent effects of the GLP-1 secretagogue of the present invention will be specifically described. Note that Examples are illustrated only for the understanding of the invention, and the present invention is not limited to these Examples. Note that before Examples are described, the result of the preliminary test is illustrated for reference.

It has been known that active GLP-1 in blood loses the physiological activity due to very rapid partial degradation by an enzyme DPP-4 and becomes inactive GLP-1. For this reason, when active GLP-1 is to be measured, it is necessary to take a countermeasure such that the blood should be stored immediately into a sampling syringe containing a peptide degradation suppressor (DPP-4 inhibitor) and measured, or a reasonable amount of blood should be collected, for example. Thus, since quantifying total GLP-1 in blood, which is a sum of active GLP-1 and inactive GLP-1, and which is not influenced by DPP-4, can find the *in vivo* secretion amount itself, total GLP-1 is basically quantified to check the effect of promoting GLP-1 secretion. Depending on the experiments, the active GLP-1 was also measured for reference.

As to GIP, total GIP, which is a sum of the active form and the inactive form, was measured.

### (Preliminary Test)

### <Cultured Cells>

A murine large intestine-derived, Glucagon-like peptide-1 (GLP-1) producing cell line GLUTag was cultured in 10%-FBS containing Dulbecco's modified Eagle's medium at 37°C in the presence of 5% CO₂.

### <GLP-1 Secretion Test>

The GLUTag cells were cultured in a 48-well plate for 2 or 3 days until subconfluence. Before each sample (D-psicose or an indigestible dextrin) was added, the wells were washed with a Hepes buffer (140 mM NaCl, 4.5 mM KCl, 20 mM Hepes, 1.2 mM CaCl₂, 1.2 mM MgCl₂, 10 mM D-glucose, 0.1% BSA, pH: 7.4). Then, 80 µl of a solution of the sample dissolved in the same buffer was added to the wells, and incubated at 37°C for 60 minutes. After the supernatant was collected, the cells were precipitated by centrifugation (800 × g, 5 minutes, 4°C), and 70 µl of the supernatant was cryopreserved. The total GLP-1 in this supernatant was measured with commercially available "Enzyme immuno assay kit" (manufactured by Yanaihara Institute Inc.).

### <Result>

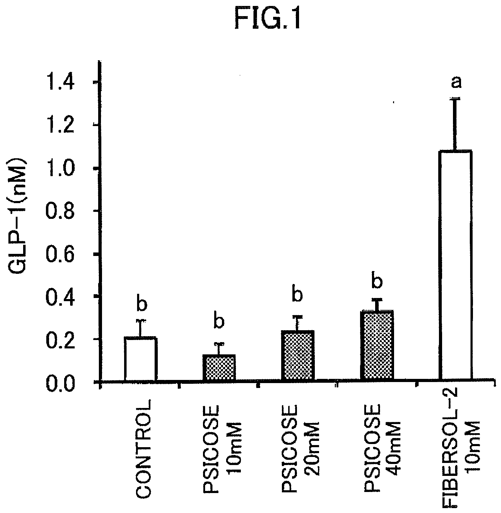

When 10 mM, 20 mM, and 40 mM solutions of D-psicose or a 10 mM solution of an indigestible dextrin (a product manufactured by Matsutani Chemical Industry Co., Ltd.: "Fibersol-2" (DE10)) were added to conduct the above-described GLP-1 secretion test, the indigestible dextrin remarkably promoted the GLP-1 secretion. In contrast, D-psicose merely tended to exhibit slight secretion promotion (Fig. 1). In other words, the effect of promoting GLP-1 secretion through the direct action on the GLP-1 producing cell line was not observed from D-psicose.

### (Example 1)

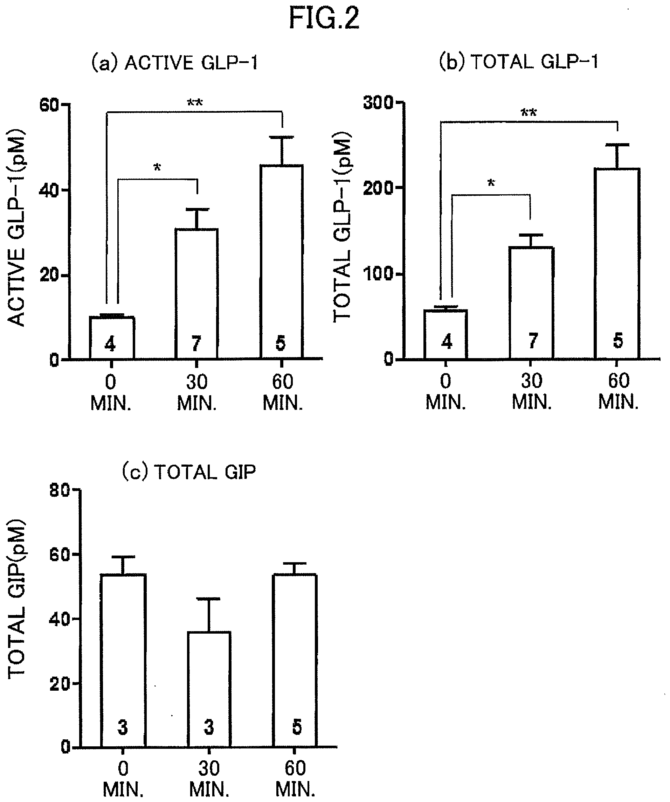

As the experimental animal, C57BL/6J male mice (9-11 weeks old) were used. To the mice having fasted for 16 hours from 18:00 on the day before the experiment, D-psicose at 1 g/kg was orally administered into each stomach at 10:00 AM. The dose of the oral administration was 10 ml/kg. Before the D-psicose administration and 30 minutes and 60 minutes after the administration, the blood was collected from the portal vein under isoflurane anesthesia. Note that an anticoagulant (heparin (final concentration: 50 IU/ml)) and peptide degradation suppressors (aprotinin (final concentration: 500 KIU/ml) and vildagliptin (final concentration: 10 µM)) had been added into the sampling syringes in advance. The collected blood was cooled, centrifuged, and stored at -80°C until the resulting blood plasma was analyzed. The quantitative analyses of active GLP-1, total GLP-1, and total GIP were conducted using ELISA kits (manufactured by Millipore Corporation. EGLP-35K, EZGLP1T-36K, and EZRMGIP-55K, respectively). In addition, as the statistical analyses, a one-way analysis of variance (paired) was performed, followed by Dunnett's test using a value before the D-psicose administration (0 min) as the control. Fig. 2 shows the result. Note that, in Fig. 2, * indicates p < 0.05, and ** indicates p < 0.01. Moreover, numerical values in bar graphs in Fig. 2 each indicate the number of experiments.

As a result, orally administering D-psicose increased the active GLP-1 and total GLP-1 concentrations in the portal veins time-dependently from 30 minutes to 60 minutes after the administration (Fig. 2 (a), (b)). In other words, it was revealed for the first time that the single oral administration of D-psicose induces GLP-1 secretion by the experiment using mice. It was speculated that D-psicose directly acts on GLP-1 producing cells (L-cells) as the mechanism of action. On the other hand, the oral administration of D-psicose did not influence the GIP secretion for 60 minutes after the administration (Fig. 2 (c)). From the foregoing, it was revealed that the single oral administration of D-psicose does not influence the secretion of GIP, which promotes lipid synthesis, and strongly induces the secretion of GLP-1 having actions such as promoting insulin secretion, suppressing appetite, and suppressing the discharge from the stomach.

### (Example 2)

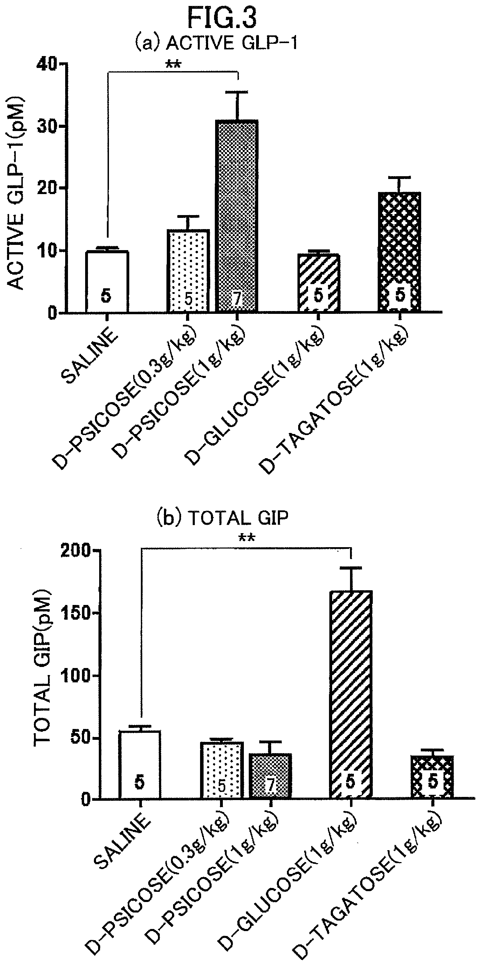

To the C57BL/6J male mice (9-11 weeks old) having fasted for 16 hours from 18:00 on the day before the experiment, D-psicose at 0.3 g/kg or 1 g/kg, D-tagatose or D-glucose each at 1 g/kg, or saline was orally administered into each stomach at 10:00 AM. The dose of the oral administration was 10 ml/kg. The blood was collected from the portal vein under isoflurane anesthesia 30 minutes after the administration. Note that an anticoagulant (heparin (final concentration: 50 IU/ml)) and peptide degradation suppressors (aprotinin (final concentration: 500 KIU/ml) and vildagliptin (final concentration: 10 µM)) had been added into the sampling syringes in advance. The collected blood was cooled and centrifuged. The blood plasma was stored at -80°C until the analysis. The quantitative analyses of active GLP-1 and total GIP were conducted using the above-described kits. In addition, as the statistical analyses, a one-way analysis of variance (unpaired) was performed, followed by Dunnett's test using saline as the control. Fig. 3 shows the result. Note that, in Fig. 3, * indicates p < 0.05, and ** indicates p < 0.01. Moreover, numerical values in bar graphs in the figure each indicate the number of experiments.

As a result, orally administering D-glucose at 1 g/kg did not change at all the active GLP-1 concentration in the portal veins for 30 minutes after the administration. On the other hand, orally administering D-psicose at 0.3 g/kg tended to increase the active GLP-1 concentration in the portal veins for 30 minutes after the administration. Orally administering D-psicose at 1 g/kg significantly increased the active GLP-1 concentration in the portal veins (Fig. 3 (a)). Moreover, orally administering D-tagatose (1 g/kg) alone tended to increase the active GLP-1 concentration in the portal veins 30 minutes after the administration. However, the value was not a significant increase in comparison with the control group, and the value was smaller than the value by D-psicose at 1 g/kg (Fig. 3 (a)). Thus, it was demonstrated that D-psicose is a GLP-1 secretagogue superior to D-tagatose. Finally, as a result of measuring the total GIP in the portal veins after D-psicose at 0.3 g/kg or 1 g/kg, D-glucose at 1 g/kg, or D-tagatose at 1 g/kg was orally administered, only the D-glucose administration group significantly increased the GIP concentration (Fig. 3 (b)). Thus, it was revealed that independent secretion promotion mechanisms exist for each of the GLP-1 and GIP secretion mechanisms by the oral administrations of the monosaccharides.

### (Example 3)

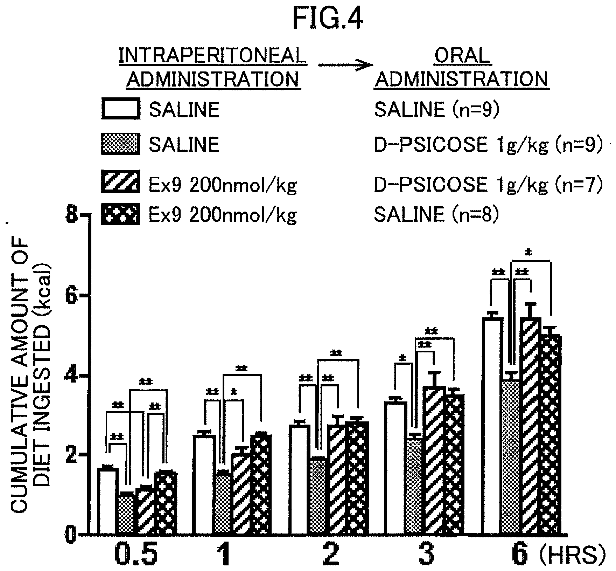

The C57BL/6J male mice (9-11 weeks old) were preliminarily grown in separate cages for one week or more, and habituated to the growth and experimental environment through training by the experimenter. After the fasting for 16 hours from 18:00 on the day before the experiment, saline or a GLP-1 receptor inhibitor (Exendin-9, Ex-9, 200 nmol/kg) was intraperitoneally administered from 9:45. Immediately thereafter, saline or D-psicose at 1 g/kg was orally administered into each stomach. The doses of the intraperitoneal administration and the oral administration were respectively 5 ml/kg and 10 ml/kg. From 10:00, the mice were fed with Diet CE-2 (common mouse diet with well-balanced nutrients, manufactured by CLEA Japan, Inc.) ad lib. After 0.5 hours, 1 hour, 2 hours, 3 hours, and 6 hours, the amounts of the diet ingested were measured over time. Each amount of the diet ingested was calculated as the amount of energy ingested (kcal), given that 1 g of D-psicose orally administered into the stomach is 0 kcal, and 1 g of Diet CE-2 is 3.45 kcal. As the statistical analyses, a one-way analysis of variance (unpaired) was performed on the results at the respective time points, and multiple comparisons were performed by Tukey's test among all the groups. Fig. 4 shows the result. Note that, in Fig. 4, * indicates p < 0.05, and ** indicates p < 0.01.

As a result, in the comparison between the "control group" (saline intraperitoneal administration and saline oral administration, white) and the "D-psicose group" (saline intraperitoneal administration and D-psicose oral administration, halftone), orally administering D-psicose at 1 g/kg significantly decreased the amount of the diet ingested from 30 minutes to 6 hours after the administration. In the "GLP-1 receptor inhibitor single administration group" (Ex9 intraperitoneal administration and saline oral administration, double hatch), the amounts of the diet ingested in all the time zones were at the same levels as those of the "control group" (Fig. 4). This suggested that endogenous GLP-1 secreted by ingesting Diet CE-2 does not influence the amount of the diet ingested.

On the other hand, in the comparison with the "GLP-1 receptor inhibitor+D-psicose group" (Ex9 intraperitoneal administration and D-psicose oral administration, hatch), the amount of the diet ingested by the "GLP-1 receptor inhibitor+D-psicose group" 30 minutes after the administration was significantly smaller than that of the "control group" or the "GLP-1 receptor inhibitor single administration group," and was at the same level as the amount of the diet ingested by the "D-psicose group." Thus, it was suggested that the ingestion suppressive action by D-psicose 30 minutes after the administration is an ingestion suppressive action not by GLP-1 (Fig. 4). The amounts of the diet ingested after 1 hour from the administration were not significantly different from the amounts of the diet ingested by the "control group" and the "GLP-1 receptor inhibitor single administration group," but significantly increased in comparison with the amounts of the diet ingested by the "D-psicose group" (Fig. 4). In other words, it was demonstrated that the ingestion suppressive action from 1 hour to 6 hours after the oral administration of D-psicose is lost by the intraperitoneal administration of the GLP-1 receptor inhibitor. From the foregoing, it was revealed that orally administering D-psicose at 1 g/kg promotes GLP-1 secretion to thereby suppress the amount of the diet ingested.

### (Example 4)

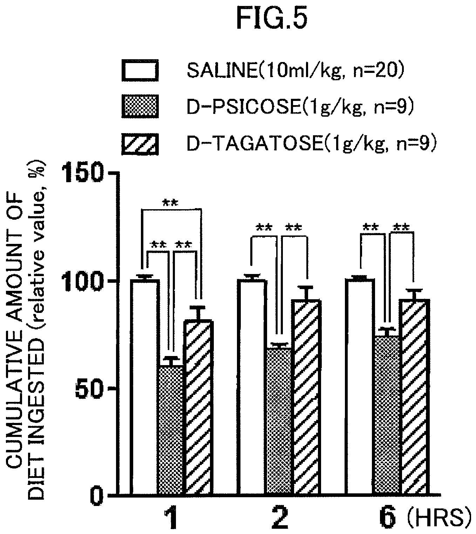

The C57BL/6J male mice (7-11 weeks old) were preliminarily grown in separate cages for one week or more, and habituated to the growth and experimental environment through training by the experimenter. After the fasting for 16 hours from 18:00 on the day before the experiment, saline, D-psicose at 1 g/kg, or D-tagatose at 1 g/kg was orally administered into each stomach from 9:50. The doses were each 10 ml/kg. From 10:00, the mice were fed with Diet CE-2 ad lib. After 1 hour, 2 hours, and 6 hours, the amounts of the diet ingested were measured over time. Each amount of the diet ingested was calculated as the amount of energy ingested (kcal), given that 1 g of D-tagatose orally administered into the stomach is 2 kcal, and 1 g of Diet CE-2 is 3.45 kcal. As the statistical analyses, a one-way analysis of variance (unpaired) was performed on the results at the respective time points, and multiple comparisons were performed by Tukey's test among all the groups. Fig. 5 shows the result. Note that, in Fig. 5, * indicates p < 0.05, and ** indicates p < 0.01.

As a result, in the "D-psicose group" (halftone), the amounts of the diet ingested from 1 hour to 6 hours after the oral administration of D-psicose were significantly smaller than those of the "control group" (saline administration, white) (Fig. 5). In the "D-tagatose administration group" (hatch), the amount of the diet ingested for 1 hour after the administration was significantly smaller than that of the "control group," but significantly larger than that of the "D-psicose group." Further, in the "D-tagatose administration group" (hatch), the amounts of the diet ingested 2 hours and 6 hours after the administration of D-tagatose were almost the same as those of the "control group," but significantly larger than those of the "psicose group." No ingestion suppressive action was recognized.

Thus, the ingestion suppressive action was recognized from the oral administration of D-tagatose at 1 g/kg, but the action lasted only in a short period of 1 hour after the administration. This revealed that the degree of the action is low in comparison with the oral administration of D-psicose at 1 g/kg. The reason of the low ingestion suppressive action by orally administering D-tagatose at 1 g/kg is presumably because the effect of promoting GLP-1 secretion is smaller than that of D-psicose (Fig. 3).

### (Example 5)

Before a sample was administered (0 minutes), the blood was collected from the tail vein of each Sprague Dawley rat (8 to 9 weeks old male) having fasted overnight. Using a feeding tube, each solution of deionized water (control group), D-psicose, an indigestible dextrin (DE10), and a dextrin (the product manufactured by Matsutani Chemical Industry Co., Ltd.: "Pinedex #2" (DE10)) was orally administered at 2 g/kg body weight (10 mL/kg body weight). Then, the blood was collected from the tail vein after 15 minutes, 30 minutes, 60 minutes, 90 minutes, 120 minutes, 150 minutes, 180 minutes, 210 minutes, and 240 minutes.

The blood was collected into a tube to which a DPP-IV inhibitor (manufactured by Millipore Corporation), aprotinin (manufactured by Wako Pure Chemical Industries, Ltd.), and heparin (manufactured by Nacalai Tesque, Inc.) had been added in advance. The blood plasma was collected by centrifugation, and cryopreserved at -80°C. The total GLP-1 concentration in the resulting blood plasma was measured with a commercially available ELISA kit (Multi Species GLP-1 Total ELISA manufactured by Millipore Corporation). The glucose concentration in the blood plasma was measured with Glucose CII-Test Wako (manufactured by Wako Pure Chemical Industries, Ltd.).

### <Test Result>

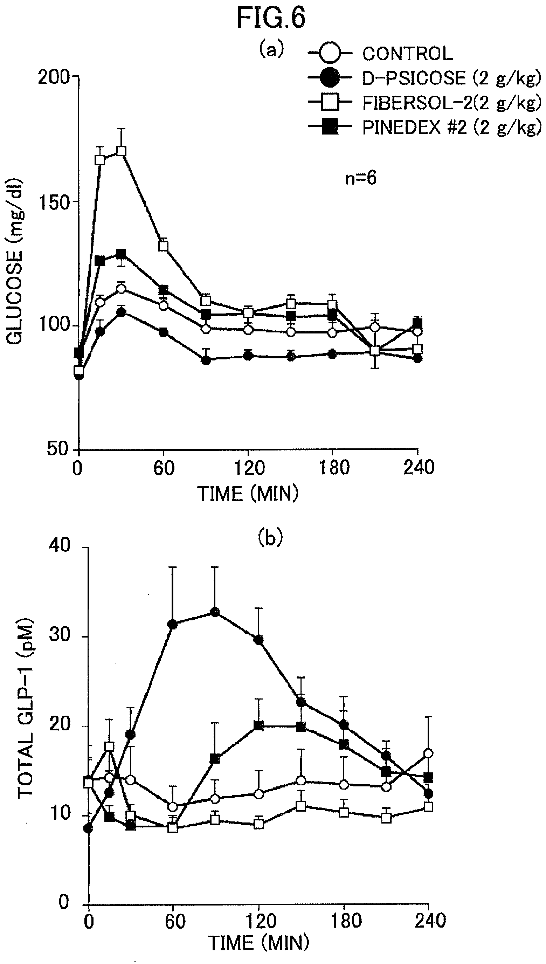

Fig. 6 shows changes in the blood sugar level and changes in the total GLP-1 concentration in the blood. The blood sugar level was increased by the dextrin administration, and slightly increased by the indigestible dextrin. A small variation was observed by the D-psicose administration as in the control group (water administration) (Fig. 6 (a)). This result confirmed that D-psicose does not have an action of increasing the blood sugar level.

On the other hand, the D-psicose administration greatly increased the GLP-1 concentration at peaks between 60 minutes and 120 minutes after the administration. Meanwhile, the indigestible dextrin administration increased the GLP-1 concentration after 90 minutes from the administration, but this action was weaker than that of D-psicose. The GLP-1 secretion was not promoted by administering the digestible dextrin (Pinedex #2) constituted of glucose and best known as a GLP-1 secretion stimulus (Fig. 6 (b)). This result showed that D-psicose has a strong action of promoting GLP-1 secretion, and that the action is persistent at the peak from 60 to 120 minutes after the administration.

### (Example 6)

### <Test Method>

After setting a termination period for one week or more in advance, 200 ml of water (control beverage) or D-psicose at 5 g/200 ml (D-psicose: 0.07 to 0.11 g/kg body weight), 10 g/200 ml (D-psicose: 0.14 to 0.22 g/kg body weight), or 15 g/200 ml (test beverage) (D-psicose: 0.21 to 0.33 g/kg body weight) each at a single dose was randomly ingested by six healthy subjects (three men and three women. The body weights: 53.3 ± 7.4 kg). In the night before the day when the control beverage or the test beverage was ingested (test day), a designated dinner was ingested. After 21:00 in the night until 9:00 AM on the test day, the ingestion of foods and beverages excluding water and tea beverages was prohibited. As the designated diet, any menu (curry, chicken and egg bowl, beef bowl, Chinese rice bowl) was selectable which hardly contain substances such as dietary fibers, lactic acid bacteria, and fermentative saccharides utilizable by intestinal bacteria for the fermentations. The blood was collected during fasting at 9:00 AM on the test day. Then, the control beverage or the test beverage was ingested. After 15 minutes, 30 minutes, 60 minutes, 120 minutes, and 180 minutes from the ingestion, the blood was collected. Note that while the blood was collected, only a designated amount of water was allowed to be ingested. The blood was collected by each subject himself or herself using a hematocrit tube into which the blood obtained by puncturing a finger tip with a cutting instrument commercially available for diabetes patients was collected. The collected blood was transferred to a microtube, and then set in a centrifuge. The total GLP-1 amount in the centrifuged blood plasma was measured using the product manufactured by Merck KGaA: "Multi Species GLP-1 Total ELISA." Note that the subjects were made to keep regular lifestyles and avoid excessive exercising, eating, and drinking during the test period of approximately one month, and spend a time quietly in house during the test.

### <Test Result>

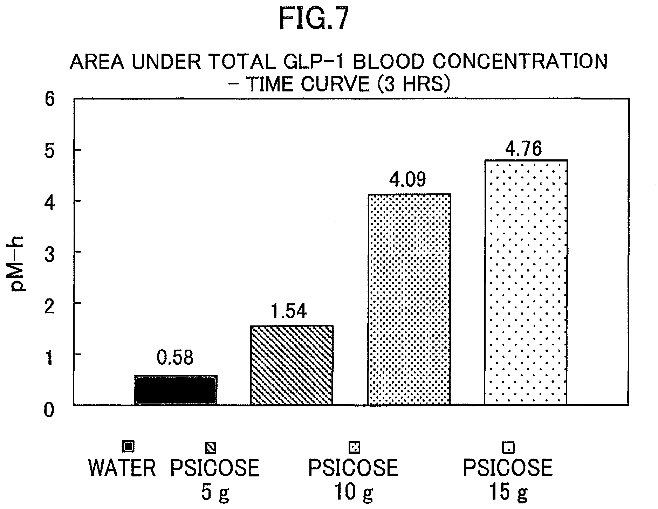

Fig. 7 shows numerical values obtained by calculating an area under total GLP-1 blood concentration - time curve (AUC) when a line graph was drawn with the vertical axis representing each total GLP-1 in blood, and the horizontal axis representing each time from 15 minutes to 180 minutes after the D-psicose ingestion. The AUC numerical value increased in a manner dependent on the amount of D-psicose ingested. It was found out that the effect of promoting the amount of total GLP-1 secreted into the blood was obtained by ingesting D-psicose at a single dose of at least 0.07 g/kg body weight.

## Claims

1. A GLP-1 secretagogue comprising D-psicose as an active ingredient.

2. The GLP-1 secretagogue according to claim 1, wherein the GLP-1 secretagogue does not comprise sucrose.

3. The GLP-1 secretagogue according to claim 1 or 2, wherein the GLP-1 secretagogue is administered during fasting.

4. The GLP-1 secretagogue according to any one of claims 1 to 3, wherein the active ingredient D-psicose is administered at a single dose of at least 0.07 g/kg body weight.

5. The GLP-1 secretagogue according to any one of claims 1 to 4, wherein the GLP-1 secretagogue is administered without ingesting sucrose or a food containing sucrose.

6. A GLP-1 secretagogue composition comprising as active ingredients:
the GLP-1 secretagogue according to any one of claims 1 to 5; and a water-soluble dietary fiber.
